# EUROPEAN PATENT SPECIFICATION

(11) **EP 2 515 094 B1**
(45) Date of publication and mention of the grant of the patent: **10.04.2019**
(21) Application number: 12152658.6
(22) Date of filing: 19.12.2003
(51) Int. Cl.: G01N 1/20, G01N 33/04, A01J 5/04, A01J 7/02, A01J 5/013, F16K 11/085, G01N 35/10

(54) **A milk conveyer device**
Vorrichtung zum Transportieren von Milch
Dispositif de transport de lait

(30) Priority: 19.12.2002 DK 200201944; 19.12.2002 US 434415 P
(43) Date of publication of application: 24.10.2012
(62) Divisional of application: 03767484.3
(73) Proprietor: Lattec I/S, 3400 Hillerod (DK)
(72) Inventor: Johannesson, Leif Börje, 146 30 Tullinge (SE); Sandberg, Ola, 646 91 Gnesta (SE); Andersson, Lars, 151 60 Södertälje (SE)
(74) Representative: Lilliehorn, Tobias

(56) References cited:
- EP-A1- 0 564 023
- DE-A- 19 547 892
- US-A- 2 821 998
- US-A- 4 722 830
- US-A1- 2002 129 668

## Description

### Field of the invention

The invention relates to a milk conveyer device and in particular to a milk conveyer device comprising functionality for analysing a characteristic of the milk.

### Background of the Invention

Milk obtained from milking of animals, such as cows or goats, has been a central product of agricultural production for many centuries. In recent decades, the use of automatic milking equipment has become predominant in industrialised countries due to the associated improvement in speed and efficiency.

In addition, it has become common place to analyse the milk from the animals in order to determine the milk quality and health of the animal. Therefore, some methods have been developed to allow for an analysis to be performed as part of an automated milking process. Specifically, analysis equipment has been developed that can chemically analyse milk samples taking from the milk produced by the milking. A chemical analysis may be performed at the site of the milking thereby allowing an individual farmer to test and analyse the milk as part of the milking process.

However, conventional milking systems are cumbersome to use in connection with the analysis equipment. For example, farmers typically have only one analysis element but may have more than one milking unit, each with milking attachment, such as a set of teat cups, for attaching to an animal to be milked. Therefore, performing an analysis typically involves disconnecting the analysis system from one milking unit, cleaning the analysis system, connecting it to another milking unit, generating a milk sample and performing the analysis. This is a very cumbersome operation, which requires significant manual intervention by the farmer. It is very time consuming, increases the workload and reduces the efficiency and productivity of the milking process.

Furthermore, insufficient cleaning results in the analysis samples being polluted thereby resulting in inaccurate or erroneous analysis results. In order to achieve sufficient cleaning, current health and hygiene standards require that the analysis equipment is thoroughly cleaned by flushing the system with significant amounts of water or of a water-based cleaning solution between analyses. Hence, conventional milking analysis systems are required to be flushed with a water-based cleaning solution to remove remnants from a previous analysis.

Systems for extracting milk samples are known from other patents such as the DE 19547892 patent wherein milk samples are extracted automatically. The milk yield being held in a collector vessel under vacuum, mixed with air when milking is over, after which a part of it is fed to a sampling vessel. The milk is delivered from the latter vessel to an analysing unit for immediate analysis. The milk remaining in the collector vessel is only extracted after analysis is complete, and is fed either to a pipe for usable milk, or to one for non-usable milk, dependent on the result. The first jets of milk when milking starts can be extracted via the pipe for non-usable milk. Before storing milk in the sampling vessel, milk can be directed via the latter into the pipe for non-usable milk, until the preceding sample is displaced.

Another example of a device for extracting milk sample is described in patent DE 3502858, which describes a device for drawing off milk samples from a delivery line, the device is used to divert milk into the sample flask by means of clocked pulses.
The object of the invention is to achieve, by means of the above mentioned device, a representative, virtually carry-over-free sample even if the delivery rate changes during a sampling operation.

A third example of a system for extracting milk samples is described in patent DE 4343717 A1, which describes a method and apparatus for taking a milk sample representative of a volume of milk. The method and apparatus minimising the risk of contamination of the milk sample by milk originating from another supply. According to the invention a major component flow of milk is branched off from a main flow and directly conducted to a mixing tank. The mixing tank is flushed by a quantity of milk before the milk branched off in a minor component flow from the main flow and conveyed to the mixing tank and collected therein.

Hence, current systems for analysing milk have a number of disadvantages including being cumbersome, wasteful and time consuming. An improved milking arrangement for analysing the milk would be advantageous.

### Summary of the Invention

Accordingly, the invention seeks to provide an improved analysing system for analysing the milk preferably alleviating or mitigating one or more of the disadvantages of the prior art singly or in combination.

It should be understood that, in any aspect of the present invention, a milking unit with a milking attachment may comprise at least one teat cup to be attached to an animal to be milked. For example, the milking unit may be implemented as a part of a so-called Voluntary Milk System which is commercially available from DeLaval International AB. The Voluntary Milk System comprises a milking robot which is ready to receive and milk a cow whenever a cow approaches the milking robot. A device with an optical system automatically cleans every teat of the cow and mounts a set of teat cups to the udder of the cow. Thereby, measurement of the milked quantity of each individual udder part is made possible. The milked quantity of each individual cow is collected before the quantity is being led to the milk storage reservoir. Alternatively, the milked quantity may be disposed if, for example, the cell count is too high, which may be an indication of mastitis. In other embodiments of the invention, the milking unit and milking attachment comprises a conventional system where teat cups are attached to the udder of the cow in a milking parlour.

According to the invention, there is provided a milk conveyer device for a milking arrangement having a milk storage reservoir; a plurality of milking units each comprising a milking attachment for attaching to an animal to be milked; a plurality of main milk conduits, each milk conduit being coupled to one of the milking attachment and the milk storage reservoir, for conveying milk from the milking attachment to the storage reservoir; the milk conveyer device comprising: an analysis element for analysing at least one selected characteristic of an analysis milk sample generated from a milk analysis quantity; a plurality of sample units, each of which being associated with one of the milking units and each of which comprises a sample element for extracting sample milk quantities from the milk of one of the main milk conduits; a plurality of sample reservoirs each of which being coupled to one of the sample elements for collecting the sample milk quantities to generate the milk analysis quantity; a plurality of analysis conduits, each of which coupled to one of the sample reservoir, for conveying the milk analysis quantity from the sample reservoirs to the analysis element; and a selector unit coupled to the plurality of analysis conduits of the milk sample units, the selector unit being operable to couple milk from one of the plurality of analysis conduits to the analysis element at a time.

According to the invention the milk conveyer device comprises a plurality of milk sample unit coupled to a selector unit operable to couple milk from either of the milk sample units to the analysis element. The selector unit may e.g. be an integral part of the analysis element or the analysis element may e.g. comprise two or more completely separate units. Hence, the selector unit and the analysis element may be two completely separate units and may be located at different physical locations. The selector unit may couple all, most, some or only a small proportion of the milk analysis quantity to the analysis element. Further, the analysis element may use only a small fraction of the received milk analysis quantity for the analysis.

The selector unit allows for a plurality of milk sample units to be permanently coupled to the analysis element and the selection of which milk sample unit to analyse samples from can be achieved by a simple control of the selector unit. This significantly facilitates the process of milking and analysing milk from a plurality of animals and thus allows for a much simpler, less cumbersome and time consuming process, as no interruption of the milking process is required.

According to another feature of the invention, the selector unit is a multi-valve unit. This provides for a suitable implementation of a selector unit.

According to another embodiment of the selector unit, it may comprise a plurality of sample stations, each sample station comprises an inlet for the milk to be tested, an outlet for the superfluous milk and an access point, accessible by a collection member for extracting a milk sample. The selector unit may further comprise at least one rinsing station for rinsing of the collection member

According to another feature of the invention, the selector unit may have access points preferably comprising covers for preventing leakage of air into the system, the cover preferably being constituted by a penetrable plug preferably being made from silicone, rubber or the like, a valve or a moveable slide cover.

According to another feature of the invention, there is provided means for generating a low pressure in a chamber of the selector unit. Preferably a pressure differential is created between the sample reservoir and the chamber of the selector unit. The pressure differential may be created by lowering the pressure of the chamber of the selector unit for example by using a pump or may e.g. be achieved through mounting the selector unit lower than the sample reservoir. The pressure differential biases the milk analysis quantity from the sample reservoir towards the selector unit and thus provides for a suitable way of establishing the flow.

According to another feature of the invention, the milk analysis element further comprises a dosage unit for generating an analysis dosage of the analysis milk sample. Typically, only a very small quantity of milk is required for the analysis and a dosage unit provides the advantage of being able to generate a suitable dose with an acceptable accuracy.

According to the invention, the selector unit comprises: a chamber, an inlet to a chamber for each of the plurality of analysis conduits for each milk sample unit, each inlet being operable to provide a flow of milk analysis quantities into the chamber, an outlet coupled to the milk analysis element, and an arm which is axially and pivotally mounted such that it is able to rotate a collection member to any of the inlets thereby providing a coupling between the inlet and the collection member which collection member is by the aid of the arm coupled to the outlet through which a part of the milk analysis quantity can be fed to the analysis element. This allows for a suitable implementation of a selector unit and specifically allows for a selector unit that can easily be controlled.

According to another feature of the invention, the selector unit of the milk analysis element is operable to collect milk of the milk analysis quantities not being coupled to the milk analysis element, or to guide such milk to the storage reservoir.

The selector unit is coupled to the milk storage reservoir whereby the collected milk is conveyed to the storage reservoir. Hence, advantageously any left over milk not coupled to the analysis element may be fed to the storage reservoir. Consequently, the left over milk is not wasted but is combined with the milk fed directly to the storage reservoir by the main milk conduit. However, it should be understood that, in alternative embodiments of the invention, any left over milk may be disposed.

These and other aspects of the invention will be apparent from and elucidated with reference to the embodiments described hereinafter.

### Brief Description of the Drawings

An embodiment of the invention will be described, by way of example only, with reference to the drawings, in which
FIG. 1 is an illustration of a milk conveyer device in accordance with a preferred embodiment of the invention.
Fig. 2 is an illustration of a sample reservoir with an element for collecting sample milk quantities into the reservoir.
FIG. 3 illustrates the milk conveyer device according to the invention implemented in a milking arrangement with a selector unit.
FIG. 4 is an illustration of a multi-valve selector unit in accordance with an embodiment of the invention.
FIGs. 5 to 10 are illustrations of elements of a milk conveyer device in accordance with an embodiment of the invention in different phases of operation.
FIG. 11 is a schematic description of a milk conveyer device in accordance with a preferred embodiment of the invention. The figure illustrates the processes for: milk collection, rinsing of the sample hose and dumping of remaining milk
FIG. 12 is a schematic description of the milk conveyer device in figure 11. The figure illustrates the process for transfer of sample to an analyse instrument.
FIG. 13 is a schematic description of the milk conveyer device in figure 11 and 12. The figure illustrates the process of cleaning the device.
FIG. 14 illustrates another embodiment of a multi-valve selector unit not part of the invention.

### Description of Preferred Embodiments

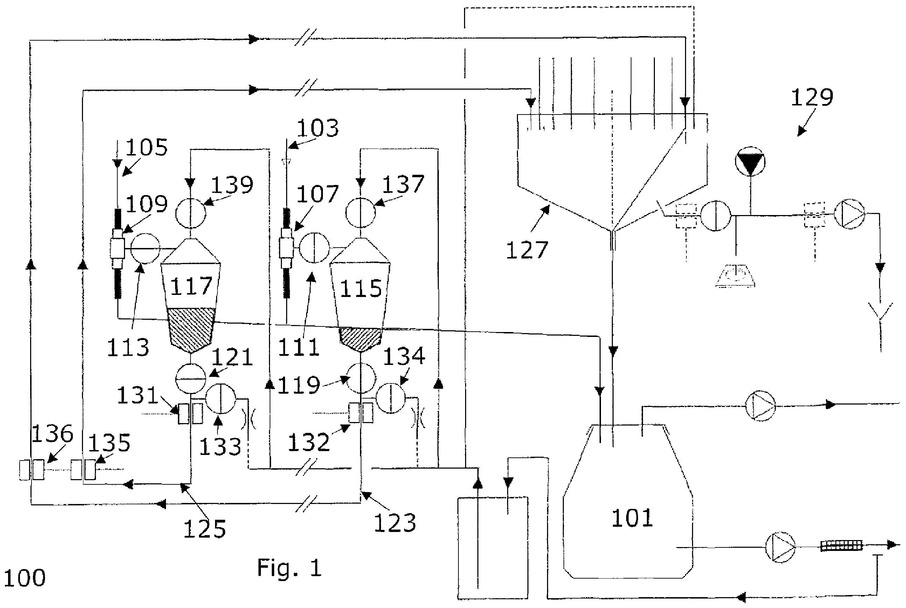

FIG. 1 is an illustration of a milk conveyer device 100 in accordance with a preferred embodiment of the invention.

The milk conveyer device is to be implemented in a milking arrangement which comprises a milk storage reservoir 101 which collects milk obtained from milking of animals. The milk is obtained from a number of milking points each of which has an associated milking unit (not shown). For clarity and brevity, FIG. 1 illustrates a specific example of two milking points but most implemented milking arrangements will typically have significantly more milking points.

Each milking unit comprises an attachment (not shown) for attaching to an animal to be milked. Such an attachment typically comprises teat cups. The attachment attaches to for example the teats of the udder of a cow, and provides a dynamic suction effect resulting in extraction of milk as is well known in the art. The milking arrangement further comprises a main milk conduit 103, 105 which guides the milk from the attachment to the milk storage reservoir wherein milk from the different milking units are collected.

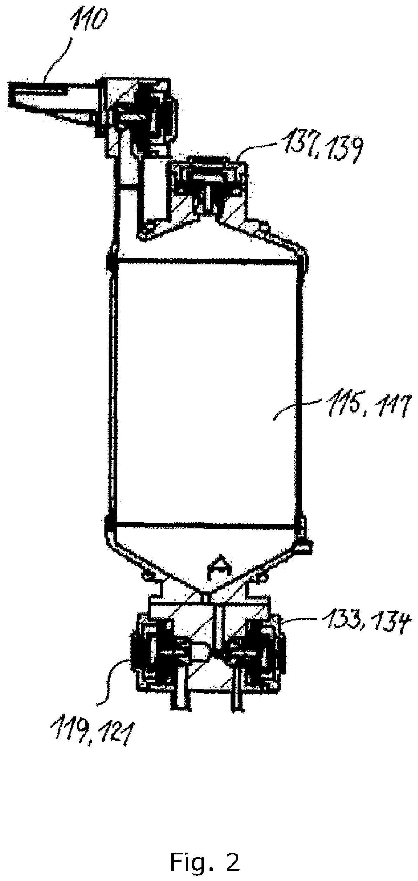

The milk conveyer device further comprises a sample element 107, 109 coupled to the main milk conduit so as to be able to extract sample milk quantities from the flow of milk in the main milk conduit. In the preferred embodiment, the sample element 107, 109 comprises a member 110 (see FIG. 2) with a small aperture extending into a cavity of the main milk conduit 103, 105 allowing for small quantities of milk to enter the aperture whereas the majority of milk bypasses the sample element to continue via the main milk conduit to the milk storage reservoir 101. Preferably the member 110 comprises an arm called knife, comprising at least one slit or aperture for receiving milk. The slit is in contact with the sample reservoir, thus the milk which has been extracted by the slit in the member 110 is preferably transferred to the sample reservoir.

Preferably the member 110 may be controlled in order to extract a representative milk sample from the milk flow in the main milk conduit. Thus during the whole milking session extract milk sample from the main milk conduit. The member may extract 2%, 4%, 6%, 8% or 10% from the milk flow in the main milk conduit. Furthermore the member 110, may be two or more members with different dimensions in order to be able to extract different amount of milk from the main milk conduit. The member 110 is designed for extracting milk from a main milk flow.

The sample elements 107, 109 may further comprise two or more members 110 for extraction of milk samples.

The aperture of the sample element is through a valve 111, 113 coupled to an sample reservoir 115, 117.The valves 111, 113 can open to allow milk to enter the sample reservoir 115, 117 or close to prevent the milk to enter the sample reservoir. Hence, the valves can be opened to sample milk flowing in the main milk conduit 103, 105 or closed to stop the sampling of milk flowing in the main milk conduit 103, 105.

The milk flowing from the sample element 107, 109 to the storage reservoir 101 flows preferably directly to the storage reservoir 101 (as indicated in Fig. 1 by the dotted lines connecting the storage reservoir 101 with the sample elements 107, 109) but may be buffered in case the result of the analysis of the milk is used as a criteria for allowing milk to flow into the storage reservoir 101. In the latter case, the milk conveyer may comprise a buffer having an outlet comprising a switch valve to guide the milk either to the storage reservoir 101 or to a waste reservoir.

The sample reservoir 115, 117 comprises a collection chamber wherein a plurality of sample milk quantities can be collected. The sample reservoir 115, 117 comprises an outlet valve 119, 121 towards the lower end of the collection chamber. The outlet valve 119, 121 is coupled to an analysis conduit 123, 125. When the outlet valve 119, 121 is closed it allows the sample milk quantities to be stored in the collection chamber 115, 117, and when the outlet valve is opened, it allows for milk to exit the collection chamber 115, 117 to enter the analysis conduit 123, 125.

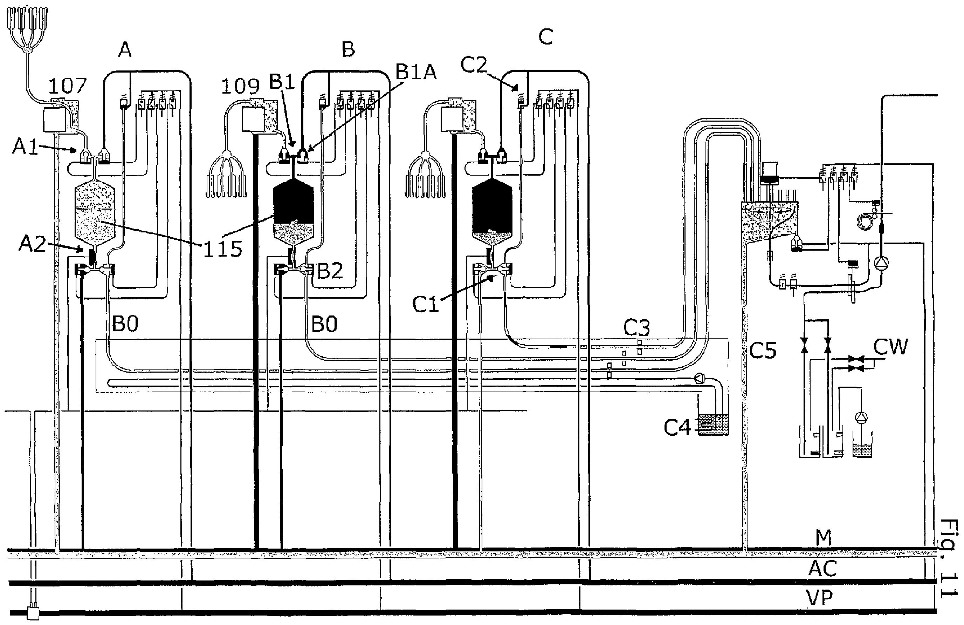

The milk is preferably mixed by a mixer in the vicinity of the bottom of the sample reservoir, preferably by utilising a membrane agitating the milk as described in figure 11.

Hence, when a milking process is started the valve 111, 113 is opened to allow sample milk quantities to be extracted from the main milk conduit and enter the collection chamber. The outlet valve 119, 121 is closed to allow the sample milk quantities to be collected in the collection chamber. During a time interval, a milk analysis quantity is collected in the sample reservoir 115, 117 from the sample milk quantities gathered during the milking process. At the end of the time interval, the valve 111, 113 is closed to prevent further milk to enter the collection chamber from the main milk conduit via the sample element 107, 109.

The collection of the sample milk quantities from the main milk conduit 103, 105 may for example be continued for a predetermined time, be manually terminated, be terminated when a suitable milk analysis quantity has been collected or be terminated when milking of an animal is completed which will cause the teat cups to drop off the udder of the cow.

When the collection of the sample milk quantities, which constitute the milk analysis quantity, from the main milk conduit 103, 105 has terminated, the outlet valve 119, 121 is opened. In the preferred embodiment, the pressure of the analysis conduit 123, 125 is lower than that in the collection chamber and consequently the milk analysis quantity will flow from the collection chamber to the analysis conduit 123, 125.

Specifically, the pressure in the analysis conduit 123, 125 is maintained at a lower pressure than atmospheric pressure and the sample reservoir 115, 117 comprises a gas inlet valve 137, 139 which can open to subject the collection chamber to a gas. The gas provides pressure to the milk analysis quantity so as to bias it towards the analysis conduit. In the preferred embodiment, the gas is an atmospheric air and the gas inlet valve simply opens to the surrounding air thereby providing atmospheric pressure. By maintaining the pressure in the analysis conduit 123, 125 below atmospheric level, this will cause the milk analysis quantity to flow into the analysis conduit 123, 125.

Each of the analysis conduits 123, 125 is coupled to a selector unit 127 through which it is possible to transfer milk from the analysis conduit 123, 125 to a milk analysis element 129.

In operation, a computer means which controls the selector unit 127 consequently selects which of the milking points are to be analysed by coupling milk from the corresponding sample element to the analysis element 129. The analysis element 129 performs a chemical analysis to determine one or more characteristics of at least part of the milk analysis quantity. Specifically, the chemical analysis may determine a concentration of urea or progesterone or any other appropriate characteristic in the milk analysis quantity. It is within the contemplation of the invention, that any suitable analysis may be performed on the milk analysis quantity in order to determine any suitable parameter.

A cleaning effect can be achieved by the milk analysis quantity in addition to the provision of conveying milk to an analysis element. Specifically, as the initial part of the milk analysis quantity when flowing through the milk conveyer device performs the function of flushing the remnants of previous milk analysis quantities. The analysis milk sample is generated from the latter part of the milk analysis quantity after the first part has cleaned the system.

The milk conveyer device is thus dimensioned such as to allow the milk analysis quantity to provide a significant cleaning effect. Specifically, the flow of the milk analysis quantity through the analysis conduit is dimensioned so as to provide the cleaning effect.

In the preferred embodiment, the analysis conduit comprises a pipe or hose having an inner cross sectional area of approximately 4 mm upstream of the selector unit and approximately 1.5 mm between the selector unit and the analysis element. The length of the pipe or hose is between 1 and 30 m. For these values, the inventors have realised that for a milk sample quantity above 200 ml and a milk analysis quantity below 100 ml sufficient cleaning of the milking system can be achieved by the milk analysis quantity. Specifically, a carry over of remnants from previous analysis processes can be reduced to less than 3% thereby providing a sufficiently small carry over to allow for a sufficiently accurate analysis to be performed.

Hence, in the preferred embodiment, no other liquid is used for cleaning the milk conveyer device between two consecutive milking processes, i.e. between two consecutive cows except for the milk analysis quantity itself, and the milk conveyer device is constructed so as to prevent that any other liquid enters the analysis conduit and the analysis element. As the only liquid present in the milk carrying parts of the system is milk, all flows can be collected in the storage reservoir without requiring any separation of the milk from other liquids. However, a water-based cleaning solution is used to clean the device following completion of milking of several animals. These animals may constitute a so-called batch.

In the preferred embodiment the control of the valves in the milk conveyer device is furthermore such that at least one gas or specifically air bubble is generated in the milk flow in the analysis conduit. This enhances the cleaning effect provided by the milk analysis quantity.

In the preferred embodiment, the selector unit 127 is as illustrated in FIG.1, coupled to the storage reservoir 101 such that the milk not being supplied to the analysis element 129 is collected in the storage reservoir 101.

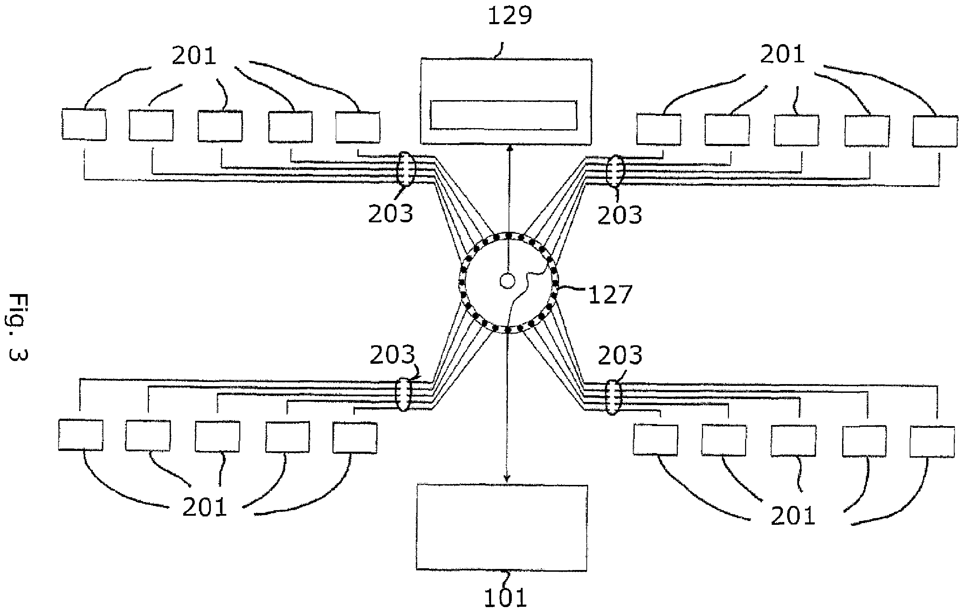

FIG. 3 illustrates the milk conveyer device according to the invention implemented in a milking arrangement with a selector unit 127. The selector unit 127 constitutes a node receiving milk through analysis conduits 203 from a plurality of milking points 201 each having an associated sample unit. Under control of the computer means, the selector unit 127 is connected to the analysis element 129 and the storage reservoir 101. The selector unit selects a single analysis conduit to be coupled to the analysis element 129 for generation of a milk sample to be analysed. Any overflow from this analysis conduit is collected and directed to the storage reservoir.

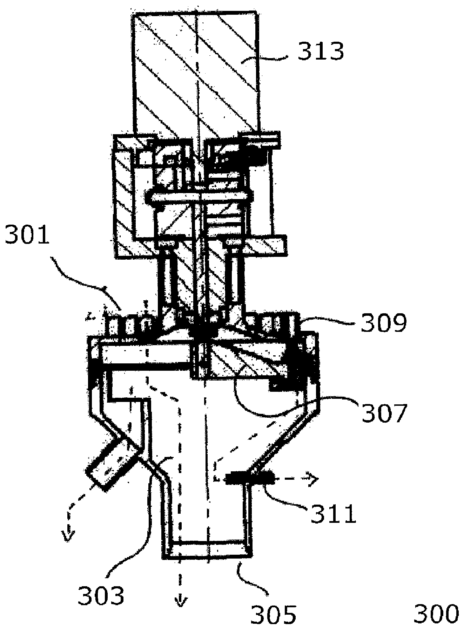

FIG. 4 is an illustration of a multi-valve selector unit 300 in accordance with an embodiment of the invention. The selector unit 300 comprises a plurality of inlets 301 to which the analysis conduits are connected. The selector unit 300 further comprises a chamber 303 into which the milk from the inlets 301 flow. The chamber has an associated outlet 305 through which the overflow milk is collected and fed to the storage reservoir. The selector unit 300 comprises a moveable member or collection element shown in the form of an arm 307. The arm 307 is axially and pivotally mounted such that it is able to rotate a collection member 309 to any of the inlets 301 thereby providing a coupling between the inlet 301 and the collection member 309. The collection member 309 is by the aid of the arm 307 coupled to an outlet 311 through which a part of the analysis quantity is fed to the analysis element. The arm 307 can be rotated by appropriate means, for example by a step motor 313 such that the coupling between one of the inlets 301 and the outlet 311 to the analysis element can be controlled by control of the step motor 313.

In the preferred embodiment, a pressure differential is created between the sample reservoirs and the selector unit to facilitate the flow from the sample reservoirs to the selector unit. In some embodiments this pressure differential may be provided by creating a reduced pressure in the chamber of the selector unit, for example by use of a pump. In other embodiments, a pressure differential is created by gravity, e.g. by the selector unit being mounted lower than the sample reservoirs.

In the preferred embodiment, the analysis element generates a suitable analysis milk sample for the analysis from the milk analysis quantity. Typically, the quantity of the analysis milk sample may be in the order of 30 to 50 ml whereas typically only 0.01 ml of milk is used for the actual analysis.

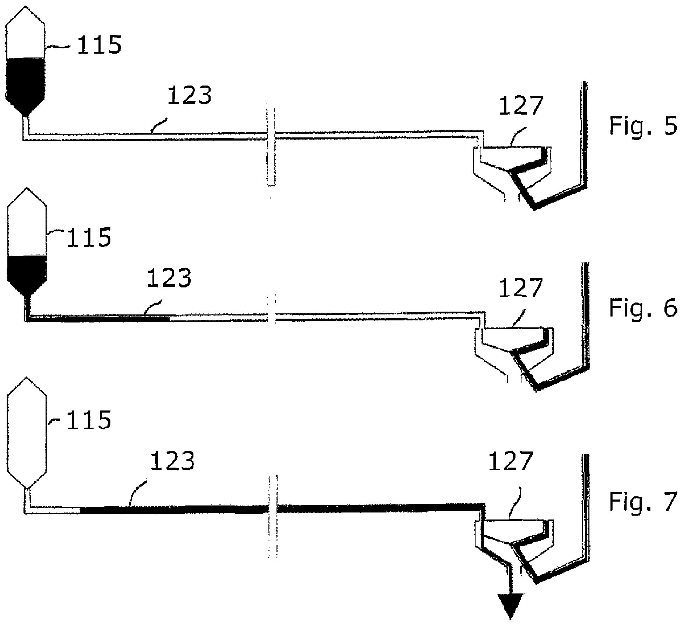

The operation of the milk conveyer device and in particular the flow of the milk analysis quantities is described in the following with specific reference to FIG. 1 and FIGs. 5 to 10. FIGs. 5 to 10 is an illustration of different components of the milk conveyer device in accordance with an embodiment of the invention in different phases of operation. Specifically, FIGs. 5 to 10 illustrate a sample reservoir 115, an analysis conduit 123 and a selector unit 127.

FIG. 5 illustrates the situation immediately prior to the outlet valve of the sample reservoir being opened. The sample process has been finished and the sample reservoir comprises the milk analysis quantity.

FIG. 6 illustrates the situation after the outlet valve 119 has been opened. Due to the pressure differential between the sample reservoir and the chamber of the selector unit, the milk analysis quantity has started to flow through the analysis conduit.

FIG. 7 illustrates the situation after the entire milk analysis quantity has left the sample reservoir. The initial part of the milk analysis quantity has reached the selector unit and is collected in the chamber and is directed to the storage reservoir. In this situation, no milk is fed to the analysis element. Rather the initial part of the milk analysis quantity is performing a cleaning function cleaning the analysis conduit and removing carry over from the previous milk analysis quantity.

In the preferred embodiment, the milk conveyer device further comprises a first sensor 131, 132 (see FIG. 1) close to the outlet valve of the sample reservoir. It is contemplated that the sensor 131, 132 may be left out e.g. as shown in FIG. 11-13. The sensor is operable to detect air bubbles in the flow in the analysis conduit. In addition, the milk conveyer device comprises a conduit inlet gas valve 133, 134 which is coupled to the analysis conduit. The coupling of the inlet gas valve to the analysis conduit is towards the outlet valve of the sample reservoir. When the sensor 131, 132 detects an air bubble indicating that the end of the milk analysis quantity has passed the sensor, the outlet valve of the sample reservoir closes such that the sample reservoir is ready to collect new milk sample quantities. At the same time the conduit inlet gas valve 133, 134 opens to provide a gas exerting a pressure on the milk analysis quantity in the analysis conduit. This causes the flow of the milk analysis quantity to continue in the analysis conduit. In the preferred embodiment, the inlet gas valve simply opens to atmospheric air.

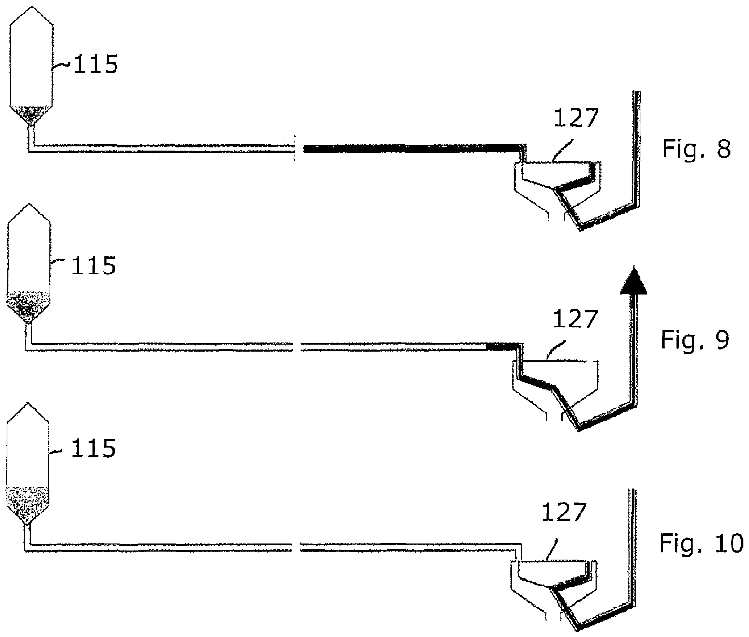

In the preferred embodiment, the milk conveyer device further comprises a second sensor 135, 136, which is located at a distance from the outlet valve further along the analysis conduit 123, 125 towards the selector unit 127. When the second sensor 135, 136 detects an air bubble indicating that the end of the milk analysis quantity has passed the second sensor 135, 136, a control mechanism closes the conduit inlet gas valve 133, 134. Following closing of the inlet gas valve 133, 134, the pressure upstream of the milk analysis quantity, i.e. the pressure behind the milk analysis quantity when seen in the flow direction, will, for a short while, remain higher than the pressure downstream of the milk analysis quantity. Thus, the milk analysis quantity continues while no air is allowed through the inlet gas valve whereby a vacuum develops upstream of the milk analysis quantity. The pressure of the upstream vacuum increases as long as the milk analysis quantity continues to flow. However, when the pressure of the upstream vacuum is equal to the pressure of the vacuum downstream of the milk analysis quantity, i.e. when the pressure differential is equalised, flow of the milk analysis quantity stops. Consequently as shown in FIG. 8 a static situation is obtained wherein a remaining part of the milk analysis quantity is left in the analysis conduit. The remaining part of the milk analysis quantity may remain in the analysis conduit until the selector unit is ready to forward it to the milk analysis element. In a specific embodiment, the second sensor 135, 136 is located such that the analysis conduit extends for approximately three to four meters from the second sensor 135, 136 to the selector unit. For the specific embodiment, this corresponds to approximately 50 ml of the milk analysis quantity remaining in the analysis conduit.

FIG. 9 illustrates the situation after the moveable arm of the selector unit has been moved to the inlet of the analysis conduit. In this situation, the appropriate inlet of the selector unit is coupled to the outlet 311 leading to the analysis element 129. The analysis element comprises a pump, which is operable to pump the remaining part of the milk analysis quantity out of the analysis conduit and into the analysis element 129 as shown in FIG. 9.

The remaining part of the milk analysis quantity, or at least a portion thereof, may be used for the analysis. Said portion of milk is called an analysis milk sample.

As illustrated in FIG. 10, when at least some of the remaining part of the milk analysis quantity has been fed to the analysis element 129, the selector unit 127 may be switched to a different inlet to provide a different analysis quantity to be transferred to the analysis element 129.

It will be clear that a milk conveyer device in accordance with the described embodiment allows for analyses to be performed for a plurality of milking points with very little effort and time consumption. Especially, very little manual control and interaction is required. Further, the milking system provides a self cleaning effect from the milk itself thereby eliminating the requirement for a separate cleaning liquid.

In the preferred embodiment, the flow through the analysis conduit 123, 125 is controlled such that the flow of the milk analysis quantities have a flow profile comprising a higher flow rate in an earlier time period and a lower flow rate in a later time period. Specifically, the initial flow rate is higher than the latter flow rate thereby providing an increased cleaning effect of the initial part of the milk analysis quantity and increased flow control for the latter part of the milk analysis quantity while providing for rapid emptying of the sample reservoirs.

The flow profile is specifically achieved by controlling the opening of one or more of the valves associated with the sample reservoir. In a preferred embodiment, the flow profile is controlled by providing a pulsating open/close signal to the conduit inlet gas valve 133, 134.

Additionally or alternatively, the conduit gas inlet valve 133, 134 is operable to be opened wider during the earlier time interval than during the second time interval.

In the preferred embodiments the sample reservoir comprises a quantity sensor for detecting a quantity of the milk analysis quantity remaining in the sample reservoir. A control mechanism is operable to open and close the valves in response to the output of this detector. Specifically, during emptying of the sample reservoir the quantity detector may detect that the remaining milk analysis quantity falls beneath a given threshold, and one or both valves may be closed to some extent thereby limiting the flow of the milk analysis quantity.

In some embodiments different flow rates are achieved by a different gas pressure applied to the milk analysis quantity through the gas inlet valve 137 and the conduit inlet gas valve 133, 134. In this way the flow rate may be reduced when the sample reservoir outlet valve is closed and the conduit inlet gas valve 133, 134 is opened.

In the preferred embodiment, the sample unit is arranged to extract sample milk quantities distributed over an extended period of a milking process. Preferably the sample milk quantities collected into a single milk analysis quantity are extracted regularly from a time towards the beginning of the milking process to a time towards the end of the milking process. Hence, preferably the sample unit performs a proportional sampling of the milk of the main milk conduit.

In the preferred embodiment, the sample reservoir further comprises a mixer for mixing the milk sample quantities. Specifically, the mixer may be a membrane mixer, which is pulsated to provide turbulence in the milk stored in the sample reservoir thereby causing a mixing effect.

FIG. 11 illustrates a preferred embodiment of a milk conveyer device according to the invention wherein the processes of milk collection, rinsing of the sample hose B0 and dumping of the remaining milk is illustrated.

During the milking process the valve A1 between the sample element 107, 109 and the sample reservoir 115 is opened. Approximate 2%, 4%, 6%, 8% or 10% of the yield is conducted from the sample element to the sample reservoir. The milk is gathered during the whole milking process to obtain a representative sample. The equipment preferably manages yields from 7 to 25 kg resulting in sample quantities preferably from 280 to 1000 ml. The A2 membrane close to the outlet of the sample reservoir is agitating the milk entering the sample reservoir in order to provide a proper mixing of the milk. When the milking process is finished, the process of rinsing of the sample hose B0 starts. The valve B1 is closed in order to close the channel to the sample element 109 that also provides vacuum for the sample reservoir. Another valve B1A at the top opens up for air inlet.

The valve B2 at the bottom of the sample reservoir is opened and milk from the sample reservoir rinses the sample hose B0 in order to reduce carry-over from previous samples. To improve the rinsing effect, rinsing milk may be mixed with small amounts of air provided from the speed valve C2.

When the preferred amount of milk sample has been extracted from the sample reservoir 115 the remaining milk from the reservoir may be dumped directly into the main milk pipeline/conduit M after which the milking equipment is ready for the next cow.
The milk sample is brought forward in the hose B0 with short intervals of air inlet from the speed valve C2 which in turn is connected to the Air/Cleaning pipeline AC. An air sensor C3 register when the last part of the milk sample passes and shuts of the speed valve C2. Thus the remaining amount of the milk sample is left inside the sample hose until the system is ready to send it to the analysis instrument.

Heating cables may be provided in order to keep the samples in temperature close to 35°C. Shown in the figure is a solution based on circulated hot water C4. However other suitable solutions such as cables etc, for keeping the milk sample warm can also be used.
The first part of the sample remaining inside the sample hose is used for rinsing of the sample hose and only the last part of milk is used for analyzing. The first part of the milk, which is used for rinsing the sample hose, may preferably be brought back to the main milk conduit M. The third main pipeline is the vacuum pipeline VP.

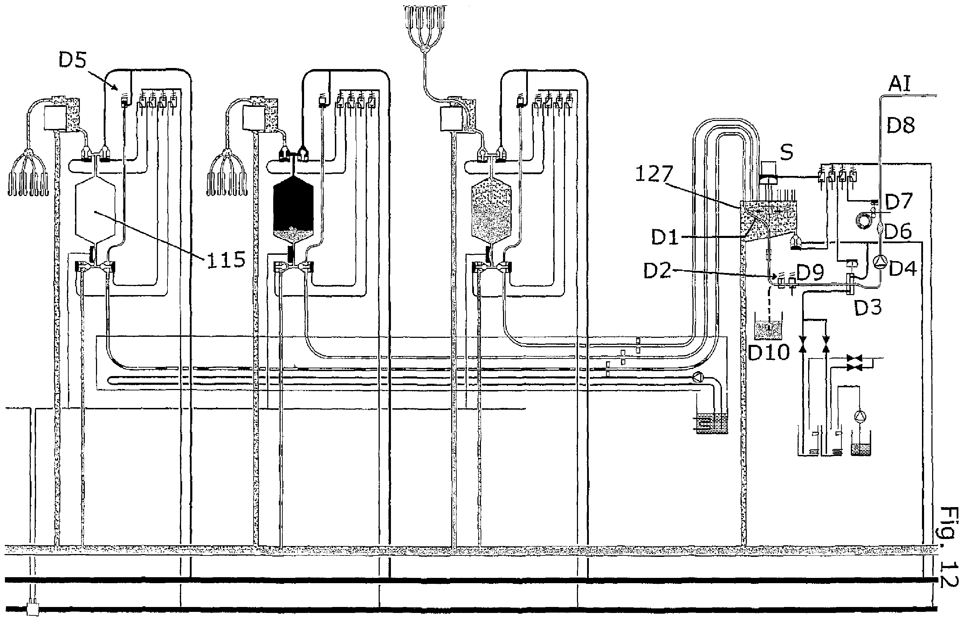

FIG. 12 illustrates the embodiment shown in figure 11, wherein the process of transferring a milk sample to an analysis instrument is illustrated.

The selector arm D1 inside the selector unit 127 is rotated by a stepper motor S to the desired position and then connected (preferably lifted with vacuum) to that samples port. Further more the shut-off valve D2 is opened and the switch valve D3 is switched from cleaning to analyzing position. The peristaltic pump D4 is activated and the milk sample is pumped towards the analyze instrument AI. Air inlet through the speed valve D5 avoids vacuum in the system. Preferably the sample reservoir 115 is disconnected from air and test hoses which admits milking of another cow to take place. Pressure shocks from the pump D4 are absorbed by a section of flexible hose D6. After that the milk sample passes through a filter D7 in order to remove particles that could damage the function of the analyze instrument. After each sample the filter material is fed forward in order to avoid jamming of the filter. When the last sample has passed the filter a number of feeding operations are performed in order to remove used filter material from the filter feeder. Preferably the filter feeder further comprises means for cutting and collection of used filter material.

The test sample is led forward inside a hose D8 and when the entire sample has passed the shut-off valve D9 (preferably time controlled), is shut off and air is provided from an adjacent electrical operated air inlet valve. D10 is a reservoir containing milk samples from cows, which have been treated with antibiotics. The milk in the reservoir D10 is preferably sucked into the system and transferred to the analysis instrument.

The hose between the selector unit 127 and the analysis instrument is preferably cleaned in the same manner as the rest of the system in order to avoid carry-over from the earlier milk sample. Thus the first part of the milk sample entering the hose provides the cleaning effect and the sample which is used in the analysis instrument is extracted from the last part of the milk sample conveyed to the analysis instrument.

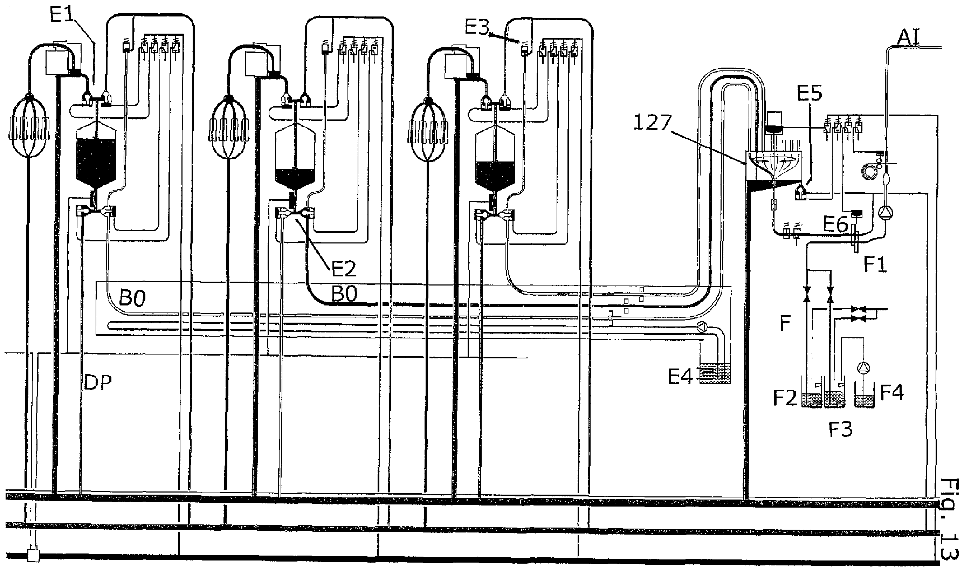

FIG. 13 illustrates the embodiment shown in figure 11-12, wherein the process of cleaning is illustrated. Cleaning of the milk conveyer device is preferably executed with a portion of cleaning liquid diverted to clean the sample container E1. The cleaning water in the sample reservoir 115 then alternates between E2 passing the dump pipe DP and the milk sample hose B0. The speed valve E3 is opened intermittent to release portions of cleaning water for rinsing the mushroom valve E2. The channel with the sample hoses is warmed up with the heating means E4, such as warm water or the alike, in order to improve the cleaning effect. A mushroom valve E5 located in the bottom part of the selector unit 127 is opened up to clean the selector unit. Cleaning water also flows through the switch valve E6 to clean the selector arm.

The hose D8 connected to the analyze instrument, is cleaned with a small cleaning unit F that can be used separate from the milking conveyer device. With the switch valve E6/F1 in cleaning mode (default mode), hose D8 is connected to the cleaning unit containers F2, F3 and F4. Detergent F4 is added to heated water in a second container F3 and pumped out for cleaning. Rinsing water is filled up, heated and pumped out through the system. Finally, the hose is rinsed with fresh water. The valve F1 is preferably a secure valve thus if it breaks it returns to a safe mode in order to avoid chemicals to enter the milk conveyer system.

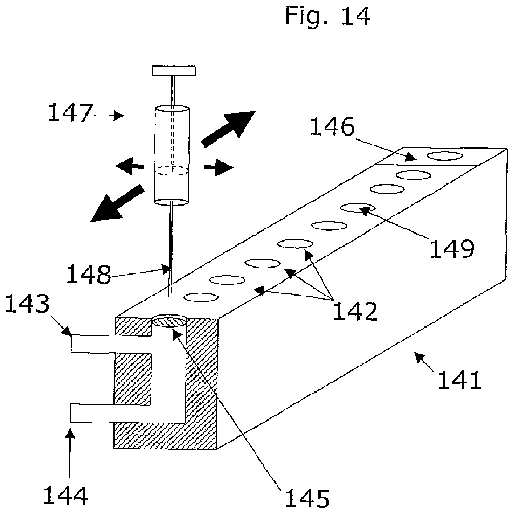

FIG. 14 illustrates a multi-valve selector unit 141 not part of the invention. The selector unit comprises a block 141 comprising a plurality of sample stations 142. Each sample station comprises an inlet 143 for the milk to be tested, an outlet 144 for the superfluous milk not used in the test and an access point 149, which can be accessed by a collection member 147 such as a needle, pipette or the like for extracting a milk sample. Each access point may be covered with a cover 145 in order to prevent leakage of air into the system. The cover may be penetrated by a part of the collection member 148.

The collection member can be moved across all the sample stations and also to a cleaning station 146 for cleaning of the collection member 147, 148. Furthermore the collection member can be moved to an application station (not shown) for application of a collected milk sample on to a test stick.

Although the present invention has been described in connection with the preferred embodiment, it is not intended to be limited to the specific form set forth herein. Rather, the scope of the present invention is limited only by the accompanying claims.

## Claims

1. A milk conveyer device (100) for a milking arrangement comprising:
- a milk storage reservoir **(101);**
- a plurality of milking units each comprising a milking attachment for attaching to an animal to be milked;
- a plurality of main milk conduits (103,105), each milk conduit being coupled to one of the milking attachments and the milk storage reservoir **(101),** for conveying milk from the milking attachments to the storage reservoir **(101);**
the milk conveyer device (100) further comprising:
- an analysis element (129) for analysing at least one selected characteristic of an analysis milk sample generated from a milk analysis quantity;
- a plurality of milk sample units, each of which being associated with one of the milking units and each of which comprises a sample element (107,109) for extracting sample milk quantities from the milk of one of the main milk conduits (103,105);
- a plurality of sample reservoirs (115,117) each of which coupled to -one of the sample elements (107,109) for collecting the sample milk quantities to generate the milk analysis
quantity;
- a plurality of analysis conduits (123,125), each of which coupled to one of the sample reservoirs (115,117) for conveying the milk analysis quantity from the sample reservoirs to the analysis element (129); and
- a selector unit (127,300) coupled to the plurality of analysis conduits (123,125) of the milk sample units, the selector unit (127,300) being operable to couple milk from one of the plurality of analysis conduits (123,125) to the analysis element (129) at a time, wherein the selector unit (300) comprises:
- a chamber (303),
- an inlet (301) to the chamber (303) for each of the plurality of analysis conduits (123,125) for each milk sample unit, each inlet (301) being operable to provide a flow of milk analysis quantities into the chamber (303), an outlet (311) coupled to the analysis element (129), and
- an arm (307) which is axially and pivotally mounted such that it is able to rotate a collection member (309) to any of the inlets (301) thereby providing a coupling between the inlet (301) and the collection member (309) which collection member (309) is by the aid of the arm (307) coupled to the outlet (311) through which a part of the milk analysis quantity can be fed to the analysis element (129).

2. A milk conveyor device (100) according to claim **1,** wherein the selector unit (127,300) is an integral part of the analysis element (129).

3. A milk conveyor device (100) according to claim **1,** wherein the analysis element (129) and the selector unit (127,300) comprise two or more completely separate units.

4. A milk conveyor device (100) according to any one of claims **1** to 3, further comprising means for generating a low pressure in a chamber of the selector unit (127,300).

5. A milk conveyor device (100) according to claim 4, wherein the means for generating a low pressure comprises a pump.

6. A milk conveyor device (100) according to claim 4, wherein the means for generating a low pressure comprises mounting the selector unit (127,300) lower than the sample reservoir (115,117).

7. A milk conveyor device (100) according to any ones of claims **1** to 6, wherein the analysis element (129) further comprises a dosage unit for generating an analysis dosage of the analysis milk sample.

8. A milk conveyor device (100) according to any one of claims **1** to 7, wherein the selector unit (127,300) is operable to collect milk of those milk analysis quantities which are not being coupled to the analysis element (129), or to guide such milk to the storage reservoir **(101).**

9. A milk conveyor device (100) according to any one of claims **1** to 8, wherein the selector unit (127,300) is coupled to the milk storage reservoir **(101)** whereby the collected milk is conveyed to the storage reservoir **(101).**

## Patentansprüche

1. Milchfördervorrichtung (100) für eine Melkanordnung, umfassend:
- einen Milchvorratsbehälter (101);
mehrere Melkeinheiten, die jeweils eine Melkaufsatz zum Anbringen an einem zu melkenden Tier umfassen;
- mehrere Hauptmilchleitungen (103; 105), wobei jede Milchleitung mit einem der Melkaufsätze und dem Milchvorratsbehälter (101) gekoppelt ist, um Milch von den Melkaufsätzen zu dem Vorratsbehälter (101) zu befördern;
wobei die Milchfördervorrichtung (100) ferner umfasst:
- ein Analyseelement (129) zum Analysieren mindestens eines ausgewählten Merkmals einer Analysenmilchprobe, die aus einer Milchanalysemenge erzeugt wurde;
- mehrere Milchprobeneinheiten, von denen jede mit einer der Melkeinheiten in Verbindung steht und von denen jede ein Probenelement (107; 109) umfasst, um Probenmilchmengen von der Milch von einer der Hauptmilchleitungen (103; 105) abzuziehen;
- mehrere Probenbehälter (115, 117), von denen jeder mit einem der Probenelemente (107; 109) zum Sammeln der Probenmilchmengen gekoppelt ist, um die Milchanalysenmenge zu erzeugen;
- mehrere Analyseleitungen (123; 125), von denen jede mit einem der Probenbehälter (115, 117) gekoppelt ist, um die Milchanalysenmenge aus den Probenbehältern (115, 117) zu dem Analyseelement (129) zu befördern; und
- Auswahleinheit (127, 300), die mit einer Mehrzahl von Analyseleitungen (123; 125) der Milchprobeneinheiten gekoppelt ist, wobei die Auswahleinheit (127, 300) betrieben werden kann, um gleichzeitig Milch von einer der Mehrzahl von Analyseleitungen (123, 125) an das Analyseelement (129) zu koppeln,
wobei die Auswahleinheit (300) umfasst:
- eine Kammer (303),
- einen Einlass (301) zu der Kammer (303) für jede der mehreren Analyseleitungen (123; 125) für jede Milchprobeneinheit, wobei jeder Einlass (301) betrieben werden kann, um einen Fluss von Milchanalysenmengen in die Kammer (303) zu gewähren, einen Auslass (311), der mit dem Analyseelement (129) gekoppelt ist, und
- einen Arm (307), der derart axial und drehbar befestigt ist, dass er in der Lage ist, ein Sammelelement (309) zu jeder beliebigen der Einlässe (301) zu drehen, wodurch ein Koppeln zwischen dem Einlass (301) und dem Sammelelement (309) gewährt wird, wobei das Sammelelement (309) mit Hilfe des Arms (307) mit dem Auslass (311) gekoppelt ist, durch den ein Teil der Milchanalysenmenge dem Analyseelement (129) zugeführt werden kann.

2. Milchfördervorrichtung (100) nach Anspruch 1, wobei die Auswahleinheit (127, 300) ein integraler Bestandteil des Analyseelements (129) ist.

3. Milchfördervorrichtung (100) nach Anspruch 1, wobei das Analyseelement (129) und die Auswahleinheit (127, 300) zwei oder mehrere vollständig separate Einheiten umfassen.

4. Milchfördervorrichtung (100) nach einem der Ansprüche 1 bis 3, ferner umfassend Mittel zum Erzeugen eines Unterdrucks in einer Kammer der Auswahleinheit (127, 300).

5. Milchfördervorrichtung (100) nach Anspruch 4, wobei das Mittel zum Erzeugen eines Unterdrucks eine Pumpe umfasst.

6. Milchfördervorrichtung (100) nach Anspruch 4, wobei das Mittel zum Erzeugen eines Unterdrucks das Anbringen der Auswahleinheit (127, 300) niedriger als den Probenbehälter (115, 117) umfasst.

7. Milchfördervorrichtung (100) nach einem der Ansprüche 1 bis 6, wobei das Analyseelement (129) ferner eine Dosierungseinheit zum Erzeugen einer Analysedosierung der Analysenmilchprobe umfasst.

8. Milchfördervorrichtung (100) nach einem der Ansprüche 1 bis 7, wobei die Auswahleinheit (127, 300) betrieben werden kann, um Milch von denjenigen Milchanalysemengen zu sammeln, die nicht mit dem Analyseelement (129) gekoppelt sind, oder um diese Milch dem Vorratsbehälter (101) zuzuführen.

9. Milchfördervorrichtung (100) nach einem der Ansprüche 1 bis 8, wobei die Auswahleinheit (127, 300) mit dem Milchvorratsbehälter (101) gekoppelt ist, wodurch die gesammelte Milch zu dem Milchvorratsbehälter (101) transportiert wird.

## Revendications

1. Dispositif de transport de lait (100) destiné à un agencement de traite comprenant :
- un réservoir de stockage de lait (101) ;
- une pluralité d'unités de traite comprenant chacune un accessoire de traite destiné à la fixation à un animal devant être traité ;
- une pluralité de conduits principaux de lait (103, 105), chaque conduit de lait étant relié à des l'un des accessoires de traite et au réservoir de stockage de lait (101), destiné au transport de lait des accessoires de traite vers le réservoir de stockage (101) ;
le dispositif de transport de lait (100) comprenant en outre :
- un élément d'analyse (129) destiné à l'analyse d'au moins une caractéristique sélectionnée d'un échantillon de lait d'analyse produit à partir d'une quantité d'analyse de lait ;
- une pluralité d'unités d'échantillon de lait, chacune desquelles étant associée à l'une des unités de traite et chacune desquelles comprenant un élément d'échantillon (107, 109) destiné à l'extraction de quantités de lait d'échantillon d'un des conduits principaux de lait (103, 105) ;
- une pluralité de réservoirs d'échantillon (115, 117), chacun desquels étant relié à l'un des éléments d'échantillon (107, 109) destiné à la collecte de quantités de lait d'échantillon pour produire la quantité d'analyse de lait ;
- une pluralité de conduits d'analyse (123, 125), chacun desquels étant relié à l'un des réservoirs d'échantillon (115, 117) destiné au transport de la quantité d'analyse de lait des réservoirs d'échantillon vers l'élément d'analyse (129) ; et
- une unité de sélection (127, 300) reliée à la pluralité de conduits d'analyse (123, 125) des unités d'échantillon de lait, l'unité de sélection (127, 300) étant utilisable pour relier du lait provenant d'un de la pluralité de conduits d'analyse (123, 125) vers l'élément d'analyse (129) à la fois,
l'unité de sélection (300) comprenant :
- une chambre (303),
- une entrée (301) vers la chambre (303) pour chacun de la pluralité de conduits d'analyse (123, 125) pour chaque unité d'échantillon de lait, chaque entrée (301) étant utilisable pour amener un flux de quantités d'analyse de lait dans la chambre (303), une sortie (311) étant reliée à l'élément d'analyse (129), et
- un bras (307) qui est monté de manière axiale et en pivot de telle sorte qu'il est capable de mettre en rotation un élément de collecte (309) à l'une quelconque des entrées (301) fournissant ainsi une liaison entre l'entrée (301) et l'élément de collecte (309), l'élément de collecte (309) étant relié, à l'aide du bras (307), à la sortie (311) à travers laquelle une partie de la quantité d'analyse de lait peut être alimentée vers l'élément d'analyse (129).

2. Dispositif de transport de lait (100) selon la revendication 1, dans lequel l'unité de sélection (127, 300) constitue une partie intégrale de l'élément d'analyse (129).

3. Dispositif de transport de lait (100) selon la revendication 1, dans lequel l'élément d'analyse (129) et l'unité de sélection (127, 300) comprennent au moins deux unités complètement séparées.

4. Dispositif de transport de lait (100) selon l'une quelconque des revendications 1 à 3, comprenant en outre un moyen de production d'une basse pression dans une chambre de l'unité de sélection (127, 300).

5. Dispositif de transport de lait (100) selon la revendication 4, dans lequel le moyen de production d'une basse pression comprend une pompe.

6. Dispositif de transport de lait (100) selon la revendication 4, dans lequel le moyen de production d'une basse pression comprend le montage de l'unité de sélection (127, 300) inférieur au réservoir d'échantillon (115, 117).

7. Dispositif de transport de lait (100) selon l'une quelconque des revendications 1 à 6, dans lequel l'élément d'analyse (129) comprend en outre une unité de dosage destinée à la production d'un dosage d'analyse de l'échantillon de lait d'analyse.

8. Dispositif de transport de lait (100) selon l'une quelconque des revendications 1 à 7, dans lequel l'unité de sélection (127, 300) est utilisable pour collecter du lait des quantités d'analyse de lait qui ne sont pas reliées à l'élément d'analyse (129), ou pour guider ce lait vers le réservoir de stockage (101).

9. Dispositif de transport de lait (100) selon l'une quelconque des revendications 1 à 8, dans lequel l'unité de sélection (127, 300) est reliée au réservoir de stockage de lait (101) par lequel le lait collecté est transporté vers le réservoir de stockage (101).
